# EUROPEAN PATENT SPECIFICATION

(11) **EP 3 790 517 B1**
(45) Date of publication and mention of the grant of the patent: **16.02.2022**
(21) Application number: 19725024.4
(22) Date of filing: 07.05.2019
(51) Int. Cl.: A61F 5/453, A61F 5/44

(54) **MALE URINARY INCONTINENCE DEVICE**
VORRICHTUNG FÜR HARNINKONTINENZ BEI MÄNNERN
DISPOSITIF POUR L'INCONTINENCE URINAIRE MASCULINE

(30) Priority: 07.05.2018 DK PA201870277
(43) Date of publication of application: 17.03.2021
(73) Proprietor: Coloplast A/S, 3050 Humlebaek (DK)
(72) Inventor: BECKER, Kim, 3400 Hilleroed (DK)
(86) International application number: PCT/DK2019/050140
(87) International publication number: WO 2019/214788

(56) References cited:
- WO-A1-87/06824
- US-A- 5 735 837
- US-A1- 2017 165 100
- US-B1- 6 209 142

## Description

The invention relates to a male urinary incontinence device.

### Brief Description of the Drawing

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Figure 1 is a cross-section view of one embodiment of a male urinary incontinence device in open position.
Figure 2 is a proximal view of one embodiment of a male urinary incontinence device.
Figure 3 is a distal view of the one embodiment of a male urinary incontinence device.
Figure 4 is an isometric view of one embodiment of a male urinary incontinence device in closed position.
Figure 5 is an isometric view of an embodiment of the male urinary incontinence device in open position.
Figure 6 is an isometric view of an embodiment of the male urinary incontinence device in closed position.
Figure 7 is an isometric view of a cuff member of an embodiment of the male urinary incontinence device.

### Detailed Description

Embodiments, and features of the various exemplary embodiments described in this application, may be combined with each other ("mixed and matched"), unless specifically noted otherwise.

In the following detailed description, reference is made to the accompanying drawings, which form a part hereof, and in which is shown by way of illustration specific embodiments in which the invention may be practiced. Because components of embodiments can be positioned in different orientations, the directional terminology is used for purposes of illustration and is in no way limiting. It is to be understood that other embodiments may be utilized and structural or logical changes may be made without departing from the scope of the present invention. Therefore, the following detailed description is not to be taken in a limiting sense, and the scope of the present invention is defined by the appended claims.

Throughout this disclosure, the words "patient" or "user" are used to address the person to wear the male urinary incontinence device. However, in some cases "user" may also relate or refer to a health care professional (HCP), such as a surgeon or continence care nurse or others. In those cases, it will either be explicitly stated, or be implicit from the context that the "user" is not the "patient" himself.

In the following, whenever referring to a proximal side of a device or part of a device, the proximal side is the side closest to the user, when the device is fitted on a user and the distal side is the opposite side - the side furthest away from the user in use.

The axial direction is defined as the direction of the penis, when the device is worn by a user. The radial direction is defined as transverse to the axial direction that is transversely to the direction of the penis. Thus, the radial direction is substantially perpendicular to the penis of the user.

The longitudinal direction is the direction from the top portion to the bottom end portion. The transverse direction is the direction perpendicular to the longitudinal direction, which corresponds to the direction across the device.

The use of the phrase "substantially" as a qualifier of certain features or effects throughout this disclosure, is intended to simply mean that any deviations are within tolerances that would normally be expected by the skilled person in the relevant field.

Many people suffer from light incontinence. Stress incontinence means that you leak urine when you sneeze, cough, laugh, lift something, change position, or do something that puts stress or strain on your bladder. Urge incontinence is an urge to urinate that is so strong that you cannot make it to the toilet in time. It also happens when your bladder squeezes when it should not. This can happen even when you have only a small amount of urine in your bladder. Overactive bladder is a kind of urge incontinence. Where the amount of leaked urine in stress incontinence may be quite small such as few drops, the amount of leaked urine in urge incontinence may be larger, such as up to 180 ml a day.

Male urinary incontinence aiding systems are known. Widely used systems known as external urinary catheters and urisheaths normally comprise a roll-on sheath or body portion for enclosing the shaft of the penis, and a tip portion that is provided with a comparatively short discharge tube, which via a tube is connected to a urine collection bag that is fastened to the leg of the user. The sheath portion is rolled-up in a number of successive windings to such an extent that adhesive on the inner surface of the sheath is accommodated in the windings. However, unrolling of a sheath correctly on a penis can be very challenging and it is essential that the sheath is fitted correctly to ensure a good sealing between the skin of the penis and the sheath. The known systems are especially intended and designed for persons suffering from medium to high urine incontinence, and the urine collection bags having a capacity of 500 ml are rather big and inconvenient to wear.

There is a need for a lightweight and discreet male urinary incontinence device that may conveniently be sealingly arranged on the penis and having a smaller capacity of about 100-300 ml and be more agreeable for the user to wear in case of lighter incontinence.

Incontinent users and health care professionals alike would welcome improvements in continence care devices to better meet such requirements.

US 6, 209,142 discloses an incontinence pouch with folded layers and an absorbent material.

US 2017/165100 discloses a urinary absorbent pouch for male incontinence having an opening in the front wall.

WO 87/06824 discloses a urine collector for male incontinence having an open side.

Embodiments relate to a male urinary incontinence device comprising a proximal wall and a distal wall, the proximal wall comprising an aperture for receiving a penis therethrough, the distal wall and the proximal wall combining to define a top section and a bottom section of the device, wherein an edge portion of the proximal wall and an edge portion of the distal wall at the bottom section of the device are sealed together to form a pocket, and wherein the proximal wall and the distal wall at the top section of the device combine to define an openable door configured to be sealingly shut to close the door in a liquid-tight/splash-proof manner, the door comprises a first lip portion and a second lip portion wherein the first lip portion and the second lip portion are concentric with the aperture . Thus, when the door is closed, the device defines a closed enclosure for the penis.

When the door is open, it facilitates easy application of the device to the penis as both sides of the aperture are accessible and the penis can be pulled through. Furthermore, by opening the door, the user can retrieve the penis from the device, without detaching the device, and urinate naturally. In embodiments, where the device is provided with an absorbent element, this can be inspected by or exchanged through the door.

In embodiments, the edge portions of the proximal wall and the distal wall at the top section of the device are configured to engage with each other to provide the liquid-tight/splash-proof seal of the door.

The edge portions of the proximal wall and the distal wall at the bottom section of the device are sealed to each other to provide a pocket for accommodating a penis.

In embodiments, the door comprises a first lip portion on the proximal wall and a second lip portion on the distal wall, the first lip portion and the second lip portion are defining the door. In embodiments, the lip portions may provide reinforcement of the door for example by being made from a more rigid material. In embodiments, the lip portions comprise a foam material. In embodiments, the lip portions are configured to seal against each other when they are superimposed.

In embodiments, the door comprises an adhesive. In embodiments, the adhesive is a foam adhesive. The adhesive may be a part of the first lip portion or the second lip portion or of both lip portions. In embodiments, the adhesive is comprised in the first lip portion, thereby decreasing the risk of the adhesive contacting the penis during application. The second lip portion may comprise release properties allowing the adhesive to be releasably fastened to the second lip portion. In embodiments, the adhesive is provided with a release liner to be removed before use to protect the adhesive during storage and application. In embodiments, the door comprises a plurality of adhesive layers, separated by a plurality of release liners. Hence, the adhesive may be renewed between each use by pulling off the used adhesive and thereby exposing the next layer of adhesive.

In embodiments, the door comprises a hook-and-loop solution such as Velcro^{®}. In embodiments, the door comprises one or more zippers.

In embodiments, the door comprises one or more tab members. The tab members provide a handle for the user to grab and facilitate easy opening of the door. In embodiments, the door comprises a lock function when it is in closed position. Such lock function may comprise a snap-lock, Velcro or adhesive. The lock function may be provided on the tab members. In embodiments, the tab members are extending from the first lip portion and the second lip portion.

In embodiments, the distal wall comprises one or more pleats. The pleat(s) may be in longitudinal direction. The pleat(s) serve(s) to provide room for the penis and allow(s) the device to enter a three-dimensional configuration. In embodiments, the distal wall comprises one pleat located centrally in longitudinal direction. In embodiments, the distal wall comprises two pleats in longitudinal direction. The two pleats provide room for the penis without the device appears bulky.

In embodiments, the distal wall and the proximal wall are flexible. In embodiments, the distal wall and/or the proximal wall comprise a film layer, such as a polymeric film. In embodiments, the distal wall and/or the proximal wall comprise a cover layer, such as a textile, a fabric or a non-woven. In embodiments, the inside surface of the walls is substantially non-absorbent. By substantially non-absorbent is herein meant that the material does not retain water/moisture, but it may in embodiments have wicking properties, leading moisture away from the surface.

In embodiments, at least the bottom end portion is provided with a liquid barrier layer. In embodiments, the pocket is provided with a liquid barrier layer. The liquid barrier layer may comprise water impermeable material. In embodiments, the pocket comprises a water impermeable film layer. In embodiments, the liquid barrier layer is in the form of a coating. In embodiments, the pocket is splash-proof. In embodiments, the device is provided with a cover layer, such as a textile or non-woven to provide a pleasant surface towards the skin of the user as well as facilitate a nice appearance. In embodiments, the cover layer is on the outside surface of the device.

In embodiments, at least a part of the inside surface of the device is provided with a skin-friendly surface, such as a non-woven. Such skin-friendly surface may provide comfort for the user as well as it may have moisture-transporting properties, securing a dry surface against the skin.

The aperture is in the top section and may in embodiments comprise a flexible cuff member to provide snug fit to penis. The cuff member provides a sufficient sealing against the shaft of the penis and enables easy application of the device. In embodiments, the cuff member is water impermeable. In embodiments, the cuff member is water impermeable but moisture permeable. In embodiments, the cuff member is splash proof.

The first lip portion and the second lip portion are concentric with the aperture. This allows sufficient space for handling the penis during application. A large door facilitates easy change of absorber and it is easy to open and close. In embodiments, the door has a width, measured radially at the broadest point, of 80-150 mm, such as 90-140 mm, such as 100-130 mm, such as 110-120 mm.

In embodiments, the pocket may comprise an absorbent element. The absorbent element is configured to absorb urine leaking from the penis. In embodiments, the absorbent element may be an integrated part of the device. This may be a suitable solution for a single use device. In embodiments, the absorbent element is a discrete element that can be exchanged and replaced with a fresh element without changing the whole device. In embodiments, the absorbent element may be provided with a handle for easy exchange of the absorbent element. Absorbent materials suitable for an absorbent element may comprise any absorbent material that is capable of absorbing and retaining fluids, such as urine.

In embodiments, the device is provided with an outlet at the bottom end portion. Such outlet may be connected, optionally via a tube or hose, to urine collecting means. In embodiments, the collecting means comprises a collecting bag. The outlet may be recloseable or it may be provided with a single use seal to be broken before use.

In embodiments, the door is reopenable and can be opened and closed several times without compromising the seal. When the door is open, it facilitates easy application of the device to the penis as both sides of the aperture are accessible and the penis can be pulled through. Furthermore, by opening the door, the user can retrieve the penis from the device, without detaching the device, and urinate naturally. In embodiments, where the device is provided with an absorbent element, this can be inspected by or exchanged through the door. In embodiments, when the door of the device is closed, the distal wall and the proximal wall are sealed along their entire edge to form a closed bag.

The aperture constitutes an opening in the device being separate from the door. Thus, the device comprises two openings: the aperture for receiving the penis and the door for aiding application, inspection and optionally allowing natural urinating.

Embodiments relate to a male urinary incontinence device comprising a top end portion, a bottom end portion, and an intermediate portion for containing a penis, the top end portion comprising an inlet through which the penis can be entered, the inlet being provided with a cuff member comprising a membrane provided with a through-going aperture and a first collar provided around the aperture, the first collar extending axially away from a first surface of the membrane, and a second collar provided around the aperture, the second collar extending axially away from a second surface of the membrane, wherein the membrane and the first and the second collar are configured to allow elastic expansion of the aperture to fittingly engage with the penis.

In embodiments, the cuff member is splash proof. In embodiments, the cuff member is waterproof.

The cuff member provides a sufficient sealing against the shaft of the penis and enables easy application of the device. In embodiments, the cuff member is water impermeable.

In embodiments, the cuff member comprises a flexible material such as an elastomer. In embodiments, the cuff member comprises a silicone material. In embodiments, the cuff member is water impermeable. In embodiments, the cuff member is water impermeable but moisture permeable.

By splash-proof is herein meant that a sample is tested to be rated to (at least) grade 3 on the Ingress Protection (IP) scale. The IP scale for moisture is shown in Table 1 below.

In embodiments, when the user is urinating (spray or splash) while wearing the device (in closed conformation) the top end portion of the device, including the door and the cuff member, should seal sufficiently enough to keep the urine inside the device. This can be tested by the below shown IPx3 scale, where water is sprayed onto the product in a 60 degree angle from vertical. During the test, water should not pass to the other side of the cuff member and the closed door in order to fulfil the IPx3 rating. When referring to "splash-proof" herein, is meant a resistance to moisture ingress rating at least 3 on the IP scale.

In embodiments, the top end portion of the device is resistant to moisture ingress by rating at least 4 on the IP scale.

In embodiments, the bottom end portion may show the same resistance to moisture ingress as the top end portion, being at least 3 on the IP scale. In embodiments, the bottom end portion is waterproof. This is tested by filling the device with water up to the cuff member (the device is held in upright position with top end portion at top). During the test the device should not leak water.

**Table 1: IP scale, second digit: Ingress of liquids**

| | |
|---|---|
| | |
| 0 | No protection. |
| 1 | Protected against vertically falling drops of water or condensation. |
| 2 | Protected against falling drops of water, if the case is disposed up to 15 from vertical. |
| 3 | Protected against sprays of water from any direction, even if the case is disposed up to 60 from vertical. |
| 4 | Protected against splash water from any direction. |
| 5 | Protected against low pressure water jets from any direction. Limited ingress permitted. |
| 6 | Protected against short periods of immersion in water. |
| 7 | Protected against long, durable periods of immersion in water. |
| 8 | Protected against close-range high pressure, high temperature spray downs. |

### Detailed Description of the Drawings

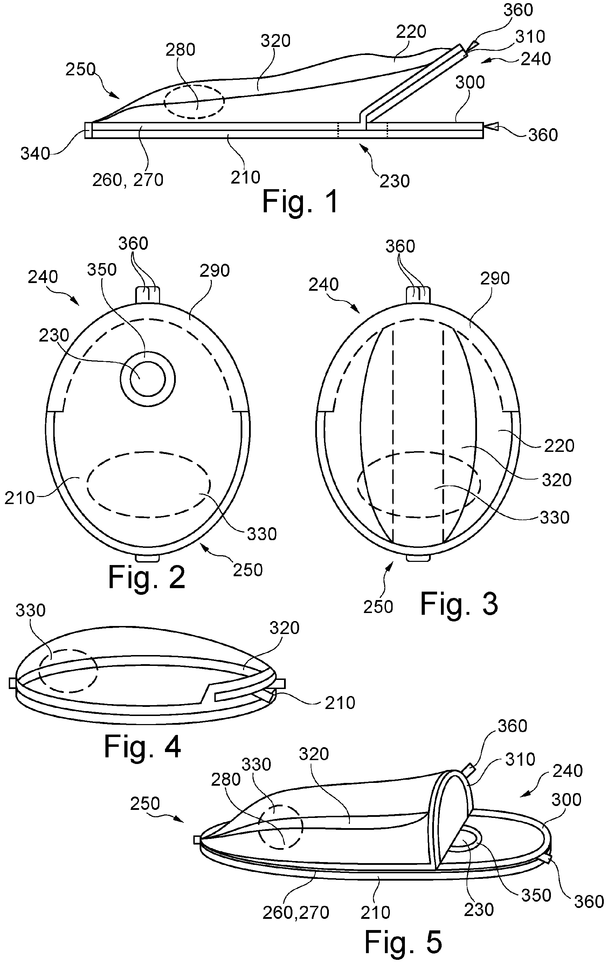

In Figures 1-5 is shown a male urinary incontinence device from different angles and in closed or open configuration. The device comprises a proximal wall 210 and a distal wall 220. The distal wall 220 and the proximal wall 210 are combined to define a top portion 240 and a bottom end portion 250 of the device, wherein an edge portion 260 of the proximal wall and an edge portion 270 of the distal wall at the bottom end portion 250 of the device are sealed together to form a pocket 280. At the top portion 240 of the device, the proximal wall 210 and the distal wall 220 combine to define an openable door 290 configured to be sealingly shut to close the door 290 in an at least splash-proof manner.

The door 290 comprises a first lip portion 300 on the proximal wall portion 210 and a second lip portion 310 and the distal wall portion 220. When the lip portions 300,310 are brought into contact with each other, the door 290 is closed and when the lip portions 300,310 are separated from each other, the door 290 is open. The first lip portion 300 may comprise an adhesive layer. The second lip portion 310 may comprise a release layer enabling the adhesive of the first lip portion 300 to fasten to it and easily release from it again when the door 290 is opened and closed. The lip portions 300,310 define a part of a circle, such as a half-circle. The door 290 is provided with tab members 360 for facilitating easy opening of the door 290. The tab members 360 may be arranged on the edge portion of the top end portion 240 of the device, on the distal wall 220 and the proximal wall 210. When the door 290 is closed, the tabs 360 may be displaced apart from each other, facilitating easy separation of the tabs 360. The tabs 360 may be a part of the lip portions 300,310, extending radially away from the device. The tabs 360 may be configured to interact with each other to form a lock when the door 290 is in closed position. In embodiments, tabs 360 are provided with Velcro or adhesive in order lock the door 290 in closed position.

The distal wall 220 may be provided with pleats 320 in longitudinal direction. The pleats 320 facilitates that the device can expand in volume to provide room for a penis and optionally an absorbent element 330. When not in use, the device may have a substantially two-dimensional (flat) configuration.

In the bottom end portion 250 of the device is optionally provided an outlet 340.

In the proximal wall 210 of the device is provided an aperture 230 for receiving a penis therethrough. A cuff 350 is surrounding the aperture 230, the cuff 350 being flexible and elastic and configured to provide sealing against the penis. The aperture 230 is located in the top end portion 240 of the device.

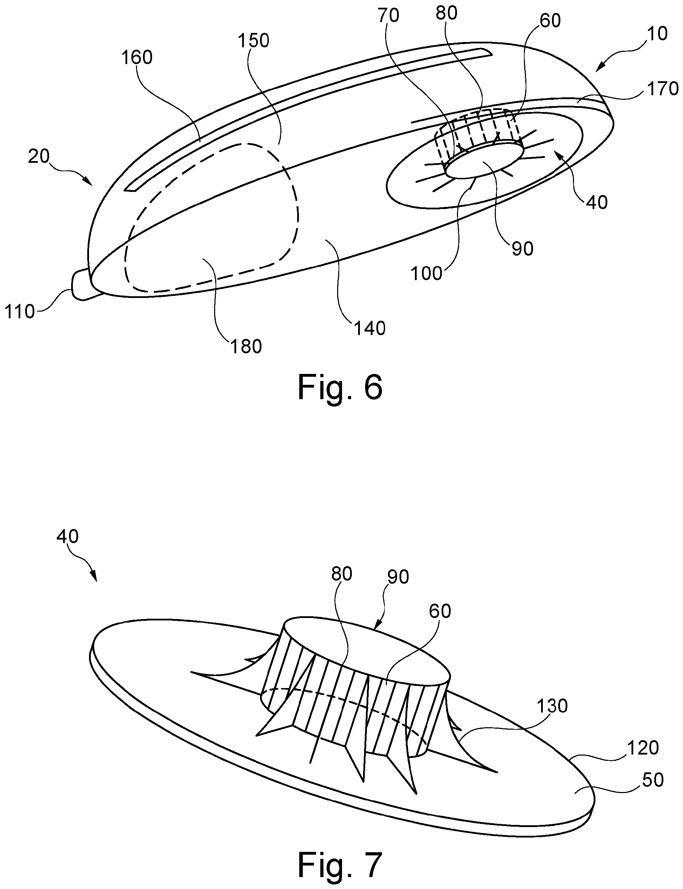

In Figure 6 is shown an embodiment of a male urinary incontinence device 10. In this embodiment, the device comprises a first wall portion 140 and a second wall portion 150, the first wall portion 140 and the second wall portion 150 being sealed along the edge portion to constitute a closed pocket. The pocket has a bottom end portion 20, optionally comprising an outlet 110, and a top end portion 10 comprising a cuff member 40. The cuff member 40 is located in the first wall portion 140. In the second wall portion 20 is provided one or more pleats 160 allowing the second wall portion 150 to expand into a three-dimensional shape, thereby providing room for accommodating at least a length of a penis. In the top end portion10 of the device, the sealing of the periphery of the first wall portion 140 and the second end portion 150 may be openable to define a closure 170. During application of the device, the closure 170 is opened, the penis is pulled through the aperture of the cuff member 40 and the closure 170 is closed again. The pocket may be equipped with an absorbent pad 180. The absorbent pad can be exchanged through the closure 170.

In Figure 7, the cuff member 40 is shown in perspective (the pocket is omitted for clarity). The cuff member 40 comprises a membrane 50 with a through-going aperture 90 and a first collar 60 and a second collar 70 provided around said aperture 90. The first collar 60 is extending in axial direction from the first surface of the membrane 50 and the second collar 70 is extending in axial direction from the second surface of the membrane 50. The first collar 60 is longer than the second collar 70. The first collar 60 may be provided with ribs 80 in the form of axially extending lines of enhanced thickness of the first collar 60. The membrane may be provided with reinforcing members 100 extending in radial direction from the aperture 90. The first collar 60 is provided with supporting wings 130 on the outside surface, the supporting wings 130 extending radially and being connected to the membrane 50. The supporting wings 130 may constitute a part of the ribs 80 of the first collar 60 and/or the reinforcing member 100 of the membrane 50 such that these structures 130,100,80 appear as a single unit.

## Claims

1. A male urinary incontinence device comprising a proximal wall (210) and a distal wall (220), the proximal wall (210) comprising an aperture for receiving a penis therethrough, the distal wall (220) and the proximal wall (210) combining to define a top end portion (240) and a bottom end portion (250) of the device, wherein an edge portion(260) of the proximal wall and an edge portion (270) of the distal wall (220) at the bottom end portion (250) of the device are sealed together to form a pocket (280), and wherein the proximal wall (210) and the distal wall (220) at the top end portion (240) of the device combine to define an openable door (290) configured to be sealingly shut to close the door (290) in a splash-proof manner, the door (290) comprises a first lip portion (300) and a second lip portion (310) **characterized in that** the first lip portion (300) and the second lip portion (310) are concentric with the aperture.

2. The device according to claim 1, wherein the edge portions (260,270) of the proximal wall (210) and the distal wall (220) at the top end portion (240) of the device are configured to engage with each other to provide the splash-proof seal of the door (290).

3. The device according to any of the preceding claims, wherein the door (290) comprises an adhesive.

4. The device according to any of the preceding claims, wherein the distal wall (220) is provided with one or more pleats (320).

5. The device according to the preceding claim, wherein the one or more pleats (320) are in longitudinal direction.

6. The device according to any of the preceding claims, wherein the distal wall (220) and the proximal wall (210) comprise flexible sheet material.

7. The device according to the preceding claim, wherein the flexible sheet material comprises fabric, textile and/or film.

8. The device according to any of the preceding claims, wherein a flexible cuff ' (350) is surrounding the aperture (230).

9. The device according to any of the preceding claims, wherein the device comprises an absorbent element (330).

10. The device according to the previous claim, wherein the absorbent element (330) comprises a handle.

11. The device according to any of the preceding claims, wherein the bottom end (250) portion of the device is liquid-tight.

12. The device according to the preceding claim, wherein the first lip portion (300) and the second lip portion (310) define a half circle.

## Patentansprüche

1. Vorrichtung für Harninkontinenz bei Männern, umfassend eine proximale Wand (210) und eine distale Wand (220), wobei die proximale Wand (210) einen Durchbruch zum Aufnehmen eines Penis dort hindurch umfasst, die distale Wand (220) und die proximale Wand (210) kombiniert werden, um einen oberen Endanteil (240) und einen unteren Endanteil (250) der Vorrichtung zu definieren, wobei ein Randanteil (260) der proximalen Wand und ein Randanteil (270) der distalen Wand (220) an dem unteren Endanteil (250) der Vorrichtung miteinander versiegelt sind, um eine Tasche (280) zu bilden, und wobei die proximale Wand (210) und die distale Wand (220) an dem oberen Endanteil (240) der Vorrichtung kombiniert werden, um eine öffnungsfähige Klappe (290) zu definieren, die ausgestaltet ist, um versiegelnd geschlossen zu werden, um die Klappe (290) spritzsicher zu verschließen, wobei die Klappe (290) einen ersten Lippenanteil (300) und einen zweiten Lippenanteil (310) umfasst, **dadurch gekennzeichnet, dass** der erste Lippenanteil (300) und der zweite Lippenanteil (310) mit dem Durchbruch konzentrisch sind.

2. Vorrichtung nach Anspruch 1, wobei die Randanteile (260, 270) der proximalen Wand (210) und der distalen Wand (220) am oberen Endanteil (240) der Vorrichtung ausgestaltet sind, um miteinander in Eingriff zu kommen, um die spritzsichere Versiegelung der Klappe (290) bereitzustellen.

3. Vorrichtung nach einem der vorhergehenden Ansprüche, wobei die Klappe (290) ein Haftmittel umfasst.

4. Vorrichtung nach einem der vorhergehenden Ansprüche, wobei die distale Wand (220) mit einer oder mehreren Falten (320) ausgestattet ist.

5. Vorrichtung nach einem der vorhergehenden Ansprüche, wobei die eine oder mehreren Falten (320) in Längsrichtung sind.

6. Vorrichtung nach einem der vorhergehenden Ansprüche, wobei die distale Wand (220) und die proximale Wand (210) flexibles Lagenmaterial umfassen.

7. Vorrichtung nach einem der vorhergehenden Ansprüche, wobei das flexible Lagenmaterial Stoff, Textilmaterial und/oder Folie umfasst.

8. Vorrichtung nach einem der vorhergehenden Ansprüche, wobei der Durchbruch (230) von einer flexiblen Manschette (350) umgeben ist.

9. Vorrichtung nach einem der vorhergehenden Ansprüche, wobei die Vorrichtung ein Absorbenselement (330) umfasst.

10. Vorrichtung nach einem der vorhergehenden Ansprüche, wobei das Absorbenselement (330) einen Griff umfasst.

11. Vorrichtung nach einem der vorhergehenden Ansprüche, wobei der untere Endanteil (250) der Vorrichtung flüssigkeitsdicht ist.

12. Vorrichtung nach einem der vorhergehenden Ansprüche, wobei der erste Lippenanteil (300) und der zweite Lippenanteil (310) einen Halbkreis definieren.

## Revendications

1. Dispositif pour incontinence urinaire masculine comprenant une paroi proximale (210) et une paroi distale (220), la paroi proximale (210) comprenant une ouverture pour recevoir un pénis à travers celle-ci, la paroi distale (220) et la paroi proximale (210) se combinant pour définir une partie d'extrémité supérieure (240) et une partie d'extrémité inférieure (250) du dispositif, une partie de bord (260) de la paroi proximale et une partie de bord (270) de la paroi distale (220) au niveau de la partie d'extrémité inférieure (250) du dispositif étant scellées ensemble pour former une poche (280), et la paroi proximale (210) et la paroi distale (220) au niveau de la partie d'extrémité supérieure (240) du dispositif se combinant pour définir une porte ouvrable (290) configurée pour être fermée de manière étanche pour fermer la porte (290) d'une manière étanche aux éclaboussures, la porte (290) comprenant une première partie de lèvre (300) et une seconde partie de lèvre (310), **caractérisé en ce que** la première partie de lèvre (300) et la seconde partie de lèvre (310) sont concentriques avec l'ouverture.

2. Dispositif selon la revendication 1, les parties de bord (260, 270) de la paroi proximale (210) et de la paroi distale (220) au niveau de la partie d'extrémité supérieure (240) du dispositif étant configurées pour venir en prise l'une avec l'autre afin de fournir le joint étanche aux éclaboussures de la porte (290).

3. Dispositif selon une quelconque des revendications précédentes, la porte (290) comprenant un adhésif.

4. Dispositif selon l'une quelconque des revendications précédentes, la paroi distale (220) étant pourvue d'un ou plusieurs plis (320).

5. Dispositif selon la revendication précédente, le ou les plis (320) étant dans le sens longitudinal.

6. Dispositif selon une quelconque des revendications précédentes, la paroi distale (220) et la paroi proximale (210) comprenant un matériau en feuille flexible.

7. Dispositif selon la revendication précédente, le matériau en feuille flexible comprenant un tissu, un textile et/ou un film.

8. Dispositif selon une quelconque des revendications précédentes, un revers souple (350) entourant l'ouverture (230).

9. Dispositif selon l'une quelconque des revendications précédentes, le dispositif comprenant un élément absorbant (330).

10. Dispositif selon la revendication précédente, l'élément absorbant (330) comprenant une poignée.

11. Dispositif selon l'une quelconque des revendications précédentes, la partie d'extrémité inférieure (250) du dispositif étant étanche aux liquides.

12. Dispositif selon la revendication précédente, la première partie de lèvre (300) et la deuxième partie de lèvre (310) définissant un demi-cercle.
